# EUROPÄISCHE PATENTSCHRIFT

(11) **EP 1 383 415 B1**
(45) Veröffentlichungstag und Bekanntmachung des Hinweises auf die Patenterteilung: **09.02.2005**
(21) Anmeldenummer: 02745271.3
(22) Anmeldetag: 26.04.2002
(51) Int. Cl.: A61B 1/00

(54) **OPTISCHES INSTRUMENT, INSBESONDERE ENDOSKOP, MIT WECHSELKOPF**
OPTICAL INSTRUMENT, PARTICULARLY AN ENDOSCOPE, WITH AN EXCHANGEABLE HEAD
INSTRUMENT OPTIQUE, NOTAMMENT ENDOSCOPE, COMPRENANT UNE TETE INTERCHANGEABLE

(30) Priorität: 27.04.2001 DE 10121450
(43) Veröffentlichungstag der Anmeldung: 28.01.2004
(73) Patentinhaber: Storz-Endoskop GmbH, 8200 Schaffhausen (CH)
(72) Erfinder: KRATTIGER, Beat, CH-8222 Beringen (CH); KUSTER, Manfred, CH-8200 Schaffhausen (CH); HAAN, Harald, CH-8200 Schaffhausen (CH)
(74) Vertreter: Heuckeroth, Volker, Dr.
(86) Internationale Anmeldenummer: PCT/EP2002/004664
(87) Internationale Veröffentlichungsnummer: WO 2002/087426

(56) Entgegenhaltungen:
- DE-U- 29 613 103
- US-A- 4 706 653
- US-A- 6 095 970
- US-A- 6 142 930
- US-B1- 6 184 923

## Beschreibung

Die Erfindung betrifft ein optisches Instrument, insbesondere Endoskop, mit einem Schaft und mit einem Wechselkopf, der mit dem distalen Ende des Schafts an einer Ankoppelstelle lösbar verbunden ist, weiterhin mit einem ersten Übertragungssystem zum Übertragen von Beleuchtungsleistung nach distal und mit einem zweiten Übertragungssystem zum Übertragen von Bildinformation nach proximal, wobei das erste Übertragungssystem und das zweite Übertragungssystem durch die Ankoppelstelle hindurchgehen, wobei das zweite Übertragungssystem eine Abbildungsoptik aufweist, wobei die Abbildungsoptik teilweise im Wechselkopf und teilweise im Schaft angeordnet ist.

Ein derartiges Instrument ist aus dem Dokument US-A-4 706 653 bekannt. Ein damit vergleichbares Instrument ist aus dem Dokument US-A-6 095 970 bekannt.

Ein derartiges optisches Instrument, insbesondere Endoskop, ist durch seine Verwendung allgemein bekannt, wobei bei bekannten Instrumenten die Wechselköpfe Wechselobjektive oder Vorsatzlinsen sind.

Obwohl die vorliegende Erfindung am Beispiel eines Endoskops beschrieben wird, läßt sie sich allgemein bei optischen Instrumenten oder Geräten einsetzen.

Endoskope werden einerseits zu medizinischen Zwecken und andererseits zu technischen Zwecken verwendet. In der Medizin werden Endoskope in der minimal-invasiven Chirurgie zur Sichtkontrolle von operativen Eingriffen verwendet. Auf technischem Gebiet werden Endoskope zur Untersuchung von schwer zugänglichen Hohlräumen, insbesondere zur Untersuchung von Arbeitsprozessen in Motoren, Turbinen und in Reaktionskammern, auch während deren Betriebs, eingesetzt.

Ferner sind unter den Endoskopen solche bekannt, deren Schaft starr ist, und solche, deren Schaft flexibel ist, wobei Endoskope mit einem flexiblen Schaft sich durch gewundene Gänge an den Einsatzort einführen lassen und außerdem mittels eines Ablenkungsmechanismus verschiedene Blickrichtungen ermöglichen.

Üblicherweise sind Endoskope mit einem ersten Übertragungssystem zum Übertragen von Beleuchtungsleistung nach distal und mit einem zweiten Übertragungssystem zum Übertragen von Bildinformation nach proximal ausgestattet. Als erstes Übertragungssystem zum Übertragen von Beleuchtungsleistung werden gewöhnlich ungeordnete Faserbündel, d.h. Lichtleiter, verwendet.

Das Übertragungssystem zum Übertragen von Bildinformation besteht bei Endoskopen entweder aus Linsen, aus geordneten Bündeln von Lichtleitfasern, d.h. sog. Bildleitern, aus elektrischen Leitungen, die von einem distal angeordneten Bildaufnehmer erzeugte elektrische Signale nach proximal leiten, aus Lichtwellen-Datenleitungen oder aus Fernübertragungen durch zeitlich modulierte Funkwellen oder zeitlich moduliertes Licht.

Am distalen Ende des Schafts des Endoskops ist üblicherweise bei bekannten Endoskopen auch eine Abbildungsoptik vorhanden, die den zu beobachtenden Raum auf den Beginn der Übertragungsstrecke, z.B. auf ein Faserbündel, abbildet.

Die verwendete Abbildungsoptik, die nicht auswechselbar ist, legt die Blickrichtung und das Gesichtsfeld fest. Um ein bestehendes Endoskop hinsichtlich Blickrichtung, Gesichtsfeld und Arbeitsabstand vielseitiger zu gestalten, werden derzeit Endoskope mit Wechselköpfen in Form von Wechselobjektiven angeboten, wobei durch die Wahl eines entsprechenden Wechselobjektivs die Blickrichtung, das Gesichtsfeld und der Arbeitsabstand verändert werden können. Auf diese Weise können die Einsatzmöglichkeiten des nicht selten sehr teuren Endoskops durch das Bereithalten verschiedener Wechselobjektive erweitert werden.

Die Wechselobjektive der bekannten Endoskope, insbesondere gemäß US-A-4 706 653 oder gemäß US-A-6 095 970, sind keine eigenständigen Optiken, sondern nur als Vorsatzlinsen mit Teleoder Weitwinkelcharakter, wie sie von der Fotografie her bekannt sind, ausgebildet. Die Wechselobjektive der bekannten Endoskope enthalten bspw. auch Umlenkungsoptiken, um von einer Geradeausblickrichtung ohne Wechselobjektiv in eine Seitblickrichtung bei angekoppeltem Wechselobjektiv umschalten zu können. Bei Wechselobjektiven der bekannten Endoskope sind zusätzlich zu dem Abbildungsoptiken ggf. noch Lichtleiter und optische Elemente für die Beleuchtung enthalten, die das Beleuchtungslicht dem Gesichtsfeld anpassen.

Um an der Ankoppelstelle den Übertritt von Beleuchtungslicht in die Beobachtungsoptik zu vermeiden, wurde bei den bekannten Endoskopen meist eine treppenartige räumliche Abstufung an der Ankoppelstelle zwischen Lichtübertragungssystem und Bildübertragungssystem vorgesehen.

Ein erhebliches Problem bei Endoskopen, die mit Wechselobjektiven ausgestattet sind, ist die Gefahr eines unbemerkten Verlusts des Wechselobjektives, wenn sich dieses vom distalen Ende des Schafts des Endoskops unerwünscht lockert. Sowohl bei Endoskopen für medizinische Zwecke als auch bei Endoskopen für technische Zwecke ist ein unbemerkter Verlust des Wechselobjektivs im Beobachtungsgebiet, d.h. bei medizinischen Endoskopen im menschlichen oder tierischen Körper, und bei technischen Endoskopen, bspw. bei einem Einsatz des Endoskops in der Flugzeugwartung bei der Triebwerksuntersuchung, unter Umständen mit verheerenden Folgen verbunden.

Bei den bekannten Endoskopen sind zur Lösung dieses Problems verschiedene Verliersicherungen wie Bajonettverschlüsse, unterteilte Gewinde, unterteilte Links-Rechtsgewinde und Gewinde-Bajonett-Kombinationen entwickelt worden.

Abgesehen davon, daß solche Verliersicherungen wegen der geringen Abmessungen der Wechselobjektive bei Endoskopen das Montieren der Wechselobjektive an den Schaft des Endoskops schwierig gestalten, lösen derartige Verliersicherungen das Problem des unbemerkten Verlustes nicht vollständig, da es beim Versagen der Verliersicherungen dennoch zu einer unbemerkten Ablösung des Wechselobjektivs vom Schaft des Endoskops kommen kann.

Wie bereits zuvor erwähnt, sind auch optische Instrumente, insbesondere Endoskope, bekannt, bei denen das Übertragungssystem zum Übertragen von Bildinformation nach proximal zumindest einen elektronischen Bildaufnehmer aufweist, der im Bereich des distalen Endes des Schafts angeordnet ist, wie beispielsweise aus US-A-5 379 756 bekannt ist. Bei diesem bekannten Endoskop ist dem nicht auswechselbaren Bildaufnehmer eine Abbildungsoptik am distalen Ende des Schafts vorgeschaltet. Dieses Endoskop weist keinen Wechselkopf auf. Aus dem Dokument US-A-6 184 923 ist ein vergleichbares optisches Instrument in Form eines Endoskops mit einem elektronischen Bildaufnehmer offenbart, wobei dieses Endoskop am distalen Ende einen Wechselkopf enthält, der eine Abbildungsoptik zum Abbilden des Beobachtungsgebietes auf den Bildaufnehmer aufweist. Bei diesem Instrument ist somit nur die Abbildungsoptik auswechselbar, nicht jedoch der Bildaufnehmer. Damit sind jedoch die Herstellungskosten des Schafts mit fest integriertem Bildaufnehmer bzw. einer Kamera erhöht.

Der Erfindung liegt die Aufgabe zugrunde, ein optisches Instrument, insbesondere Endoskop, der eingangs genannten Art dahingehend weiterzubilden, daß die Gefahr eines unbemerkten Verlustes des Wechselkopfs vermieden wird.

Erfindungsgemäß wird diese Aufgabe hinsichtlich des eingangs genannten optischen Instruments dadurch gelöst, daß der Wechselkopf und/oder die Ankoppelstelle derart ausgebildet ist, daß bei einer Lockerung des Wechselkopfs eine qualitativ wahrnehmbar veränderte Bildinformation übertragen wird, und daß der im Schaft angeordnete Teil der Abbildungsoptik auswechselbar ist.

Die erfindungsgemäße Lösung beruht auf dem Prinzip, dem Benutzer bereits eine Lockerung des Wechselkopfs, die zu einem vollständigen Ablösen vom Schaft des Endoskops führen kann, dadurch anzuzeigen, daß sich das beobachtete Bild bei einer Lockerung des Wechselkopfes qualitativ wahrnehmbar verändert. Eine solche Veränderung des beobachteten Bilds kann sich darin äußern, daß das Bild heller und kontrastschwächer, dunkler, schlechter bzw. unschärfer wird oder gar verschwindet. Die Art und Weise, wie sich die übertragene Bildinformation bei einer Lockerung des Wechselkopfes ändert, hängt davon ab, auf welchem Prinzip die Übertragung der Beleuchtungsleistung und/oder die Übertragung der Bildinformation basiert, wie sich aus der nachfolgenden Beschreibung von bevorzugten Ausgestaltungen der Erfindungen ergibt. Die erfindungsgemäßen Lösungen lösen sich somit von dem Konzept, lediglich eine Verliersicherung an der Ankoppelstelle zwischen dem Wechselkopf und dem Schaft des Endoskops vorzusehen. Bei den bekannten Endoskopen kann eine Lockerung des Wechselkopfs vom Schaft des Endoskops dagegen nicht aufgrund der übertragenen Bildinformation bemerkt werden, da die Wechselköpfe der bekannten Endoskope lediglich als Vorsatzlinsen ausgebildet sind. Bei den bekannten Endoskopen bleibt nämlich die Bildschärfe und der Kontrast bei sich lockerndem oder abgefallenem Wechselobjektiv erhalten, es ändert sich nur leicht das Gesichtsfeld, was kaum wahrnehmbar ist, wenn man nicht ausschließlich darauf achtet. Bei unerwartetem Lösen bleibt die Änderung des Gesichtsfeldes unbemerkt, da sie die Arbeit nicht beeinflußt.

Die Abbildungsoptik des erfindungsgemäßen Instruments ist teilweise im Wechselkopf und teilweise im Schaft angeordnet, wobei der im Schaft angeordnete Teil der Abbildungsoptik auswechselbar ist.

Bei dieser Ausgestaltung, bei der das zweite Übertragungssystem ein optisches System darstellt, wird eine Lockerung des Wechselkopfes dem Benutzer durch eine Verschlechterung des Bildes, d.h. durch ein verschwommenes Bild, angezeigt. Bei einem Verlust des Wechselkopfes kann das Bild dann wieder vollständig verschwinden. Die teilweise Aufteilung der Abbildungsoptik auf den Wechselkopf und auf den Schaft hat den Vorteil, daß der Wechselkopf vereinfacht und mit geringeren Kosten realisiert werden kann, was insgesamt zu einer Kostenreduzierung des optischen Instruments führt, da ein Teil der Abbildungsoptik nur einmal vorgesehen werden muß, nämlich im Schaft des Instruments.

Die Auswechselbarkeit des im Schaft angeordneten Teils der Abbildungsoptik hat den Vorteil, daß das Endoskop bei Bedarf auf eine neue zukünftige Wechselkopf-Palette umgerüstet werden kann.

In einer bevorzugten Ausgestaltung umfaßt das erste Übertragungssystem einen Lichtleiter, der sich durch den Schaft und durch den Wechselkopf erstreckt und an der Ankoppelstelle unterbrochen ist, und weisen das distale Ende des Schafts und der Wechselkopf an der Ankoppelstelle jeweils eine ebene, vorzugsweise polierte Fläche auf.

Bei dieser bevorzugten Ausgestaltung macht sich die Lockerung des Wechselkopfes durch eine Aufhellung und damit einhergehende Trübung (Kontrastschwächung) der übertragenen Bildinformation bemerkbar, wenn das zweite Übertragungssystem zum Übertragen der Bildinformation ein Bildleitersystem auf der Basis von Fasern oder Linsen basiert. Während bei bestehenden Wechselköpfen in Form von Wechselobjektiven eine treppenartige Abgrenzung der eng zusammenliegenden Lichtübertragungs- und Bildübertragungssysteme zur gegenseitigen Trennung vorliegt, wurde herausgefunden, daß ebene polierte Grenzflächen durch Beugung und Absorption eine gewisse kleine Spaltbreite (ungleich Null) unter einer kritischen Spaltbreite zulassen, ohne daß eine Bildverschlechterung eintritt. Die Herstellungstoleranzen erlauben es, bei festgezogener Verbindung zwischen dem Wechselkopf und dem distalen Ende des Schafts die Spaltbreite unter dieser kritischen Spaltbreite zu halten. Erst bei einer Lockerung des Wechselkopfes, d.h. bei sich vergrößerndem Spaltabstand, wenn die kritische Spaltbreite überschritten wird, tritt dieser Effekt der Bildbeeinträchtigung auf. Kommt es dann zu einer Lockerung des Wechselkopfs, wird aufgrund dieser Maßnahme wegen der Spaltvergrößerung zwischen Wechselkopf und Schaft Beleuchtungslicht in das Bildübertragungssystem reflektiert, wodurch der Benutzer durch die Bildtrübung eine Lockerung des Wechselkopfs erfährt und gewarnt wird. Ein weiterer Vorteil dieser Maßnahme besteht darin, daß durch die passende Auflage zwischen dem Wechselkopf und dem Schaft zusätzlich der Innenraum gegen Eintreten von Flüssigkeiten und Stäuben abgedichtet ist. Des weiteren läßt sich die flache Auflagefläche zwischen Wechselkopf und Schaft einfacher herstellen als die bei herkömmlichen Endoskopen vorgesehene treppenartig abgestufte Auflage zwischen dem Wechselkopf und dem Schaft.

Die zuvor genannten Ausgestaltungen, bei denen das zweite Übertragungssystem eine Abbildungsoptik aufweist, sind bevorzugt auch bei solchen optischen Instrumenten verwendbar, bei denen das zweite Übertragungssystem einen elektronischen Bildaufnehmer aufweist, der im Schaft des Instruments angeordnet ist. Bei einer Lockerung des Wechselkopfes kommt es zu einer Störung der Abbildung eines Objekts auf den Bildaufnehmer, so daß auch in diesem Fall ein verschlechtertes Bild bemerkt wird, sobald sich der Wechselkopf lockert. Auch ist die Abbildungsoptik bei einer solchen Ausgestaltung wiederum teilweise im wechselkopf und teilweise im Schaft vor dem Bildaufnehmer angeordnet.

In einer weiteren bevorzugten Ausgestaltung weist das erste Übertragungssystem eine Lichtquelle und eine elektrische Energieleitung von proximal zur Lichtquelle auf, wobei die Lichtquelle im Wechselkopf angeordnet ist, und ist die Ankoppelstelle so ausgebildet, daß die Signalleitung bei einer Lockerung des Wechselkopfs nach distal unterbrochen ist.

Bei dieser Ausgestaltung basiert das erste Übertragungssystem ähnlich wie bei der zuvor genannten Ausgestaltung ebenfalls auf einer elektrischen Energieübertragung, wobei die Lichtquelle dann im Wechselkopf, beispielsweise in Form einer LED, angeordnet ist. Bei einer Lockerung des Wechselkopfes kann eine Unterbrechung der elektrischen Energieleitung auftreten, indem beispielsweise im Bereich der Ankoppelstelle entsprechende Kontakte zwischen dem Wechselkopf und dem distalen Ende des Schafts vorgesehen sind.

In einer weiteren bevorzugten Ausgestaltung ist der Wechselkopf mit dem distalen Ende des Schafts an der Ankoppelstelle mittels zumindest eines Positionierstifts, der in eine entsprechende Bohrung eingreift, verbunden.

Hierbei ist von Vorteil, daß die Wechselköpfe beim Ankoppeln an den Schaft genau positioniert werden können, damit Bildschärfe und Bildausrichtung stimmen. Es kann ein unrunder Positionierstift mit einer entsprechend komplementären Bohrung ausreichen, um diesen Zweck zu erfüllen. Bevorzugt sind jedoch zumindest zwei Positionierstifte, wodurch der Wechselkopf noch genauer zentriert und außerdem exakt verdrehgesichert werden kann. Außerdem eröffnen die Positionierstifte die zusätzliche Möglichkeit, für eine elektrische Signal- bzw. Energieübertragung genutzt zu werden. Bei den bekannten Endoskopen wird eine Verdrehsicherung des Wechselobjektivs durch eine Indexnute am Umfang des Schafts bzw. des Wechselobjektivs erreicht, wobei die Zentrierung durch eine genaue Durchmesserpassung erreicht wird. Somit sind zur Positionierung des Wechselobjektivs bei den bekannten Endoskopen zwei unabhängige Elemente erforderlich, die jeweils mit der nötigen Präzision eingearbeitet werden müssen. Dies ist jedoch aufwendig bei der Herstellung, da komplizierte Maschinen und Arbeitsprozesse erforderlich sind.

In einer weiteren bevorzugten Ausgestaltung ist der zumindest eine Positionierstift auswechselbar.

Hierbei ist von Vorteil, daß bei einer Beschädigung, bspw. Verbiegung des Positionierstifts, nur dieser ausgetauscht werden muß, ohne daß der Wechselkopf als Ganzes unbrauchbar wird. Demgegenüber besteht bei den bekannten Endoskopen, bei denen die Wechselobjektive mittels Nocken und Indexnuten verdrehgesichert sind, die Gefahr, daß die Nocken beim Richten abbrechen und das Wechselobjektiv als Ganzes unbrauchbar wird.

In einer weiteren bevorzugten Ausgestaltung dient der zumindest eine Positionierstift zur elektrischen Signal- oder Energieübertragung.

Die Verbindung des Wechselkopfs mit dem Schaft des Endoskops über den zumindest einen Positionierstift und die Bohrung kann vorteilhafterweise derart ausgebildet sein, daß bei einer Lokkerung des Wechselkopfs die elektrische Signalübertragung durch die Ankoppelstelle hindurch unterbrochen ist.

In Verbindung mit der erfindungsgemäßen Ausgestaltung, daß der Benutzer über eine Lockerung des Wechselkopfs durch eine Verschlechterung des übertragenen Bildes gewarnt ist, haben die zuvor erwähnten Positionierstifte den weiteren Vorteil, daß die Positionierstifte die Verbindung zwischen dem Wechselkopf und dem Schaft noch aufrechterhalten können, während bereits der Benutzer über die Lockerung des Wechselkopfs gewarnt ist. Mit anderen Worten wird der Benutzer über eine Lockerung und der Gefahr eines Abfallens des Wechselkopfs bereits gewarnt, während der Wechselkopf mit dem Schaft des Endoskops über die Positionierstifte noch haltend in Verbindung steht.

In einer weiteren bevorzugten Ausgestaltung ist zwischen dem Wechselkopf und dem distalen Ende des Schafts zumindest ein elastisches Element angeordnet, das bei einer Lockerung des Wechselkopfs diesen vom distalen Ende des Schafts beabstandet.

Ein solches elastisches Element zwischen Schaft und Wechselkopf, beispielsweise eine Feder oder ein Elastomer, bewirkt, daß bei sich lockerndem Wechselkopf stets ein vergrößerter Abstand und somit die herbeizuführende Bildbeeinträchtigung entsteht. Damit kann vorteilhafterweise verhindert werden, daß sich möglicherweise die Überwurfmutter zwischen dem Wechselkopf und dem distalen Ende des Schafts lockert, daß dies aber durch den ggf. noch eng anliegenden Kontakt zwischen dem Wechselkopf und dem distalen Ende des Schafts nicht bemerkt werden kann.

Dabei ist es weiterhin bevorzugt, wenn die Beabstandung auf weniger als die Länge des zumindest einen Positionierstifts begrenzt ist.

Durch diese Maßnahme kann im Zusammenhang mit der Verliersicherung vorteilhafterweise vermieden werden, daß das elastische Element bei einer Lockerung des Wechselobjektivs den Wechselkopf vollständig von dem distalen Ende des Schafts abstößt und der Wechselkopf dadurch verlorengeht. Die Begrenzung kann beispielsweise durch einen Anschlag oder dadurch realisiert werden, daß das elastische Element im entspannten Zustand eine Längsausdehnung aufweist, die bezüglich des distalen Endes des Schafts geringer ist als die Länge des zumindest einen Positionierstifts, so daß der Wechselkopf durch den Positionierstift noch am Schaft gehalten ist. ,

In einer weiteren bevorzugten Ausgestaltung ist in dem Wechselkopf zumindest ein Arbeitselement angeordnet.

Mit dieser Ausgestaltung können dem Wechselkopf neben der Funktion der Bild- und Lichtübertragung zusätzliche Funktionen zugeordnet werden, beispielsweise kann das zumindest eine Arbeitselement Hilfsfunktionen für die Abbildung wie Fokussierung, Zoom, Blickrichtungsänderung, Bildrotation, aktive und passive Sensorfunktionen beispielsweise für Abstand, Druck, Temperatur, Vibrationen, Oberflächenhärte, Ultraschallreflektion, Schichtdicke, Dielektrizitätskonstante, magnetische Permeabilität, Brechungsindex, induzierte Wirbelströme, elektrische Magnetfelder, Radar, Lidar, ionisiernde Strahlung, Wärmekapazität, elektrische und thermische Leitfähigkeiten, pH-Werte, chemische Stoffkonzentrationen, Feuchte, Fluoreszenz, optische Rückstreuung aus der Tiefe, usw. erhalten, ebenso wie Bearbeitungsfunktionen wie Greifen, Entnehmen, Schleifen, Bohren, Sägen, Wasserstrahlbearbeiten, Sandstrahlen, Abblasen mit Gasen, Heizen, Kühlen, Laserbearbeiten, usw. In diesem Sinne ist "Arbeitselement" ganz allgemein zu verstehen.

Dabei ist bevorzugt, wenn das zumindest eine Arbeitselement mittels eines Steckers mit dem distalen Ende des Schafts verbindbar ist.

Diese Ausgestaltung ist insbesondere bei Arbeitselementen, die auf eine elektrische Stromversorgung angewiesen sind, vorteilhaft, da dann auch die Stromzuführung für das zumindest eine Arbeitselement an der Ankoppelstelle über den Stecker trennbar und leicht zusammenfügbar ist.

Weitere Vorteile ergeben sich aus der nachfolgenden Beschreibung und der beigefügten Zeichnung.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Bevorzugte Ausführungsbeispiele der Erfindung sind in der Zeichnung dargestellt und werden in der nachfolgenden Beschreibung näher erläutert. Es zeigen:
- Fig. 1: ein Endoskop mit einem Wechselkopf in einer perspektivischen Gesamtdarstellung gemäß einem ersten Ausführungsbeispiel;
- Fig. 2a) und b): das Endoskop in Fig. 1 im Bereich der Ankoppelstelle zwischen dem Wechselkopf und dem distalen Ende des Schafts des Endoskops in einer getrennten Darstellung, wobei Fig. 2a) den Wechselkopf und Fig. 2b) das distale Ende des Schafts zeigt,
- Fig. 3: einen Längsschnitt durch den Wechselkopf mit einer teilweisen Aufbrechung in einer gegenüber Fig. 2a) verdrehten Stellung;
- Fig. 4: einen Längsschnitt durch das distale Ende des Schafts in Fig. 2b);
- Fig. 5: eine Vorderansicht des distalen Endes des Schafts des Endoskops;
- Fig. 6a) und b): den Wechselkopf und das distale Ende des Schafts im Längsschnitt, wobei Fig. 6a) einen Zustand zeigt, in dem der Wechselkopf vom distalen Ende des Schafts des Endoskops gelockert ist, und Fig. 6b) einen Zustand, in dem der Wechselkopf fest mit dem distalen Ende des Schafts verbunden ist;
- Fig. 7: ein distales Ende eines optischen Instruments gemäß einem weiteren Ausführungsbeispiel mit einem Wechselkopf in einer schematischen Seitenansicht in einen Zustand, in dem der Wechselkopf vom distalen Ende des Schafts gelöst ist;

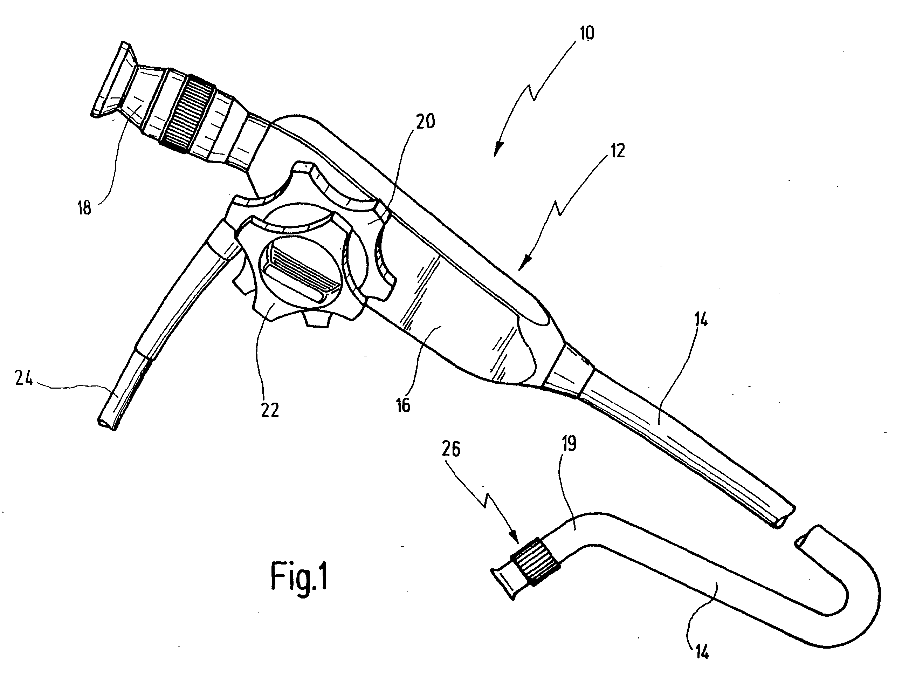

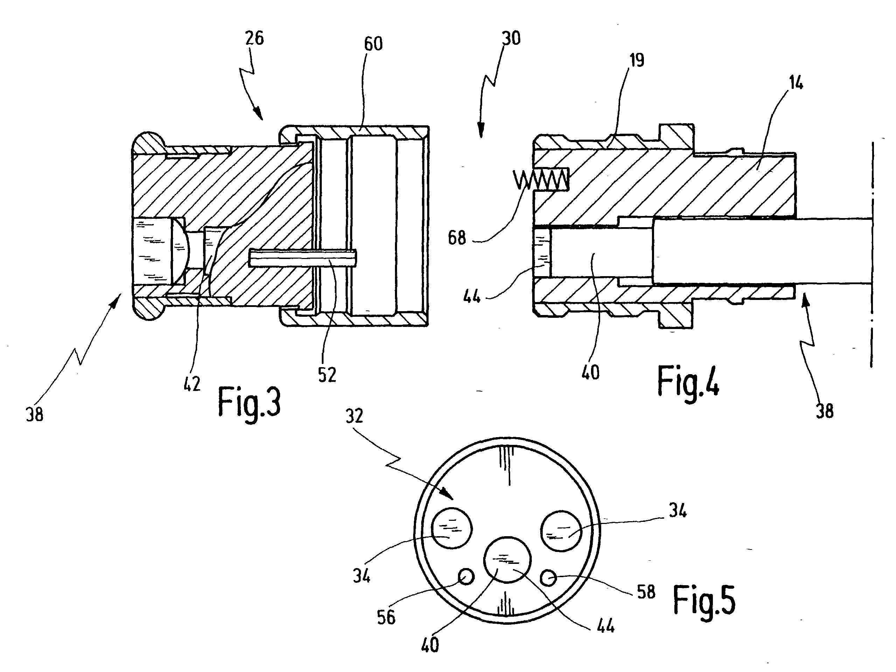

In Fig. 1 ist ein mit dem allgemeinen Bezugszeichen 10 versehenes optisches Instrument dargestellt. In dem gezeigten Ausführungsbeispiel ist das optische Instrument 10 ein Endoskop 12, ohne daß die Erfindung auf ein solches Instrument beschränkt ist.

Das Endoskop 12 weist einen langerstreckten Schaft 14 auf. Der Schaft 14 ist in dem hier gezeigten Ausführungsbeispiel flexibel, derart, daß der Schaft 14 außer einem geraden auch einen gekrümmten Verlauf einnehmen kann, wie in Fig. 1 beispielhaft dargestellt ist.

Das Endoskop 12 wird als technisches Endoskop bei der Inspektion von Maschinen, bspw. Flugzeugtriebwerken, verwendet. Das Endoskop 12 kann auch im Rahmen der minimal-invasiven Chirurgie zu Operationszwecken eingesetzt werden.

Am proximalen Ende des Schafts 14 weist das Endoskop 12 ein Bedienteil 16 auf. Am proximalen Ende des Bedienteils 16 ist ein Okular 18 angeordnet, das Teil eines später noch näher beschriebenen Übertragungssystems des Endoskops 12 zum Übertragen von Bildinformation darstellt. Durch das Okular 18 kann mit dem Auge direkt beobachtet werden, oder es kann an das Okular 18 eine nicht dargestellte Kamera angeschlossen werden, wobei dann die Kamera an ein Bildwiedergabegerät, bspw. einen Monitor, angeschlossen wird, auf dem das mit dem Endoskop 12 beobachtete Gebiet visuell dargestellt wird.

Das Endoskop 12 ist wie bei flexiblen Endoskopen üblich mit einem Ablenkmechanismus zum Ablenken eines distalen Endes 19 des Schafts 14 ausgestattet. Der Ablenkmechanismus umfaßt einen nicht näher dargestellten Mechanismus, der über ein erstes Stellrad 20 und ein zweites Stellrad 22 am Bedienteil 16 betätigbar ist.

Der Ablenkmechanismus arbeitet in zwei unabhängigen Ablenkrichtungen, wobei das erste Stellrad 20 und das zweite Stellrad 22 jeweils einer Ablenkrichtung zugeordnet sind.

Am Endoskopgehäuse 16 ist ferner ein Lichtleitkabel 24 angeschlossen, das mit einer nicht dargestellten externen Lichtquelle zum Zuführen von Beleuchtungslicht in das Endoskop 12 verbunden ist.

Mit dem distalen Ende 19 des Schafts 14 ist ein Wechselkopf 26 an einer Ankoppelstelle 30 lösbar verbunden.

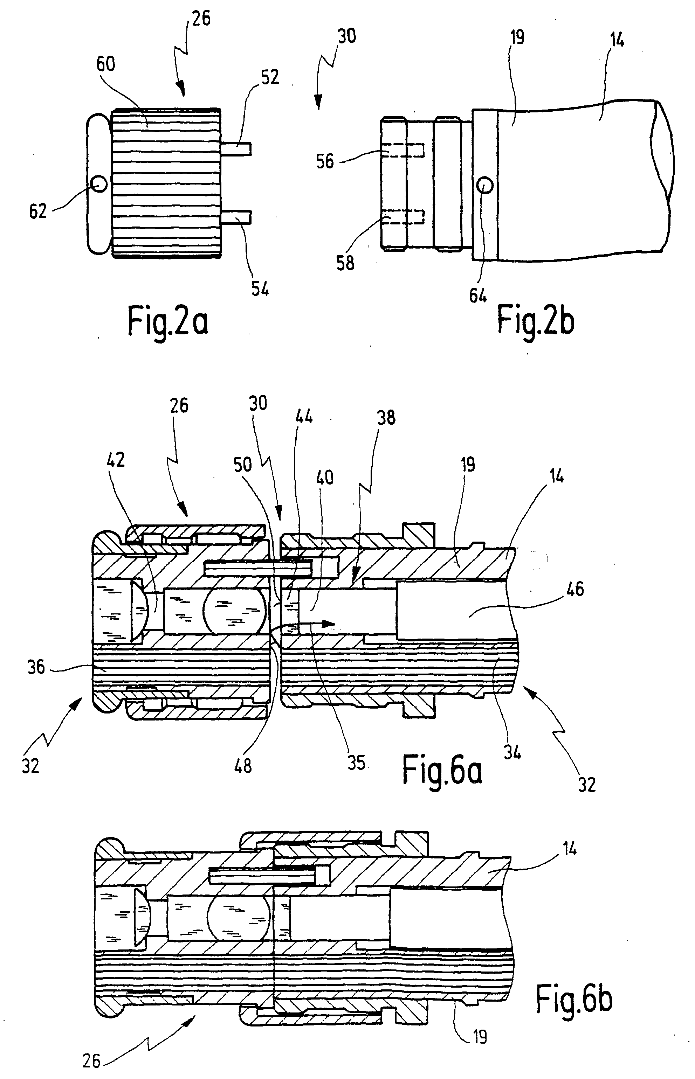

In Fig. 2 bis 6 sind Einzelheiten des Wechselkopfs 26 und der Ankoppelstelle 30 dargestellt.

Das Endoskop 12 weist ferner ein Übertragungssystem 32 zum Übertragen von Beleuchtungsleistung auf, das hier aus zwei Faserbündeln 34 gebildet ist, die sich parallel vom distalen Ende 19 des Schafts 14 durch den Schaft 14 und das Lichtleitkabel 24 hindurch bis zu dem nicht dargestellten Anschlußstecker zum Anschließen des Lichtleitkabels 24 an eine Lichtquelle (nicht dargestellt) erstrecken. Das Übertragungssystem 32 zum Übertragen von Beleuchtungsleistung beruht somit im vorliegenden Ausführungsbeispiel vollständig auf der Übertragung von Lichtwellen.

Das Übertragungssystem 32 weist ebenso zwei Faserbündel 36 auf, die sich durch den Wechselkopf 26 durchgehend erstrecken und im mit dem distalen Ende 19 des Schafts 14 verbundenen Zustand des Wechselkopfs 26 mit den Faserbündeln 34 zusammenwirken, d.h. durch die Ankoppelstelle 30 hindurchgehen.

Das Endoskop 12 weist weiterhin ein Übertragungssystem 38 zum Übertragen von Bildinformation auf, das hier aus einem geordneten Faserbündel 40 besteht, das sich durch den Schaft 14 vom distalen Ende 19 bis zum proximalen Ende des Endoskopgehäuses 16, d.h. bis zum Okular 18, erstreckt. Das Übertragungssystem 38 beruht im vorliegenden Ausführungsbeispiel ebenfalls vollständig auf der Übertragung von Lichtwellen.

Das Übertragungssystem 38 weist ferner eine Abbildungsoptik 42 auf, die im Wechselkopf 26 angeordnet ist, und im angekoppelten Zustand des Wechselkopfs 26 die Bildinformation in das geordnete Faserbündel 40 des Schafts 14 einkoppelt.

Die Abbildungsoptik 42 des Wechselkopfs 26 besteht aus einer Hintereinanderschaltung verschiedener Linsen, die in Fig. 6a) und 6b) durch entsprechend gekrümmte Flächen veranschaulicht sind.

Das geordnete Faserbündel 40 des Schafts 14 ist an seinem distalen Ende mit einem planparallelen Deckglas 44 geschützt. Das Deckglas 44 hat keine optisch abbildende Wirkung. Die gesamte Abbildungsoptik 42 des Endoskops 12 ist im Wechselkopf 26 angeordnet, so daß bei vollständig abgenommenem Wechselkopf 26 wie in Fig. 2a) und b) und Fig. 3 und 4 durch das Endoskop 12 keine Bildinformation übertragen wird.

Es kann die Abbildungsoptik 42 aber auch in einer alternativen Ausführung, die hier nicht dargestellt ist, teilweise im Wechselkopf 26 und teilweise im Schaft 14 angeordnet sein, wobei dann der im Schaft 14 angeordnete Teil der Abbildungsoptik 42 vorzugsweise auswechselbar ist.

Das geordnete Faserbündel 40 ist ferner mit einem Schutzmantel 46 überzogen.

Aufgrund der vollständigen Anordnung der Abbildungsoptik 42 im Wechselkopf 26, während der Schaft 14 kein derartiges optisch abbildendes System aufweist, verschlechtert sich die Qualität der durch das Übertragungssystem 38 übertragenen Bildinformation bereits bei einer geringfügigen Lockerung des Wechselkopfs 26 von dem distalen Ende 19 des Schafts 14. Dieser Zustand ist in Fig. 6a) dargestellt. Bei einer wie in Fig. 6a) auftretenden übermäßigen Spaltbildung infolge einer Lockerung des Wechselkopfs 26 nimmt die durch das Okular 18 beobachtbare Bildschärfe deutlich ab, wodurch dem Benutzer des Endoskops 12 eine Lockerung des Wechselkopfs 26 angezeigt wird.

An der Ankoppelstelle 30 weisen der Wechselkopf 26 und das distale Ende 19 des Schafts 14 ferner ebene und vorzugsweise polierte Flächen 48 (Wechselkopf 26) und 50 (distales Ende 19 des Schafts 14) auf.

Bei einer Lockerung des Wechselkopfs 26 tritt, wie in Fig. 6a) dargestellt ist, Beleuchtungslicht 35 aus den Faserbündeln 34 durch Reflexionen an der Fläche 48 des Wechselkopfs 26 in das geordnete Faserbündel 40 des Übertragungssystems 38 ein, so daß der Benutzer bei einer derartigen Lockerung des Wechselkopfs 26 ein deutlich helleres Bild wahrnimmt.

Bei ordnungsgemäß vollständig mit dem distalen Ende 19 des Schafts 14 verbundenen Wechselkopf 26, wie in Fig. 6b) dargestellt ist, tritt dagegen kein Beleuchtungslicht von dem Übertragungssystem 32 in das Übertragungssystem 38 über. Eine Kontrastminderung des durch das Okular 18 beobachteten Bildes tritt jedoch bei einer Vergrößerung des Spaltes zwischen dem Wechselkopf 26 und dem distalen Ende 19 auf, sobald sich der Spalt über ein kritisches Minimum hinaus vergrößert, so daß der Benutzer auch durch die Trübung bzw. Aufhellung des Bildes über eine Lockerung des Wechselkopfs 26 gewarnt ist.

Die Flächen 48 und 50 sind, wie bereits erwähnt, vorzugsweise flach poliert.

Ferner ist der Wechselkopf 26 mit dem distalen Ende 19 des Schafts 14 über zwei Positionierstifte 52 und 54, die am Wechselkopf 26 befestigt sind, an der Ankoppelstelle 30 verbunden, wobei im distalen Ende 19 des Schafts 14 entsprechende Bohrungen 56 und 58 ausgespart sind.

Die Positionierstifte 52 und 54 dienen im Zusammenwirken mit den Bohrungen 56 und 58 einerseits zur exakten Positionierung des Wechselkopfs 26 relativ zum distalen Ende 19 des Schafts 14, damit die Abbildungsoptik 42 des Wechselkopfs 26 mit dem geordneten Faserbündel 40 exakt zusammenwirkt. Dadurch wird eine exakte Bildausrichtung und Bildschärfe gewährleistet.

Zum anderen bewirken die Positionierstifte 52 und 54 im Zusammenwirken mit den Bohrungen 56 und 58 eine Verdrehsicherung des Wechselkopfs 26 am Schaft 14.

Die Positionierstifte 52 und 54 halten auch bei einer bereits erfolgten Lockerung, die in Fig. 6a) dargestellt ist, noch eine Verbindung mit dem distalen Ende 19 des Schafts 14 aufrecht. Wenn der Benutzer, wie zuvor beschrieben, über die Lockerung des Wechselkopfs 26 bereits durch eine Verschlechterung der Bildqualität, d.h. eine auftretende Unschärfe und/oder durch eine Trübung bzw. Aufhellung des Bildes gewarnt ist, bleibt der Wechselkopf 26, wenn auch lose, noch mit dem Schaft 14 verbunden, so daß das Endoskop zusammen mit dem Wechselkopf 26 noch rechtzeitig aus dem Beobachtungsgebiet zurückgezogen werden kann, bevor sich der Wechselkopf 26 vollständig ablöst.

Schließlich ist der Wechselkopf 26 mittels einer an dem Wechselkopf 26 unverlierbar gesicherten Überwurfmutter 60, die auf dem Wechselkopf 26 im nicht angekoppelten Zustand des Wechselkopfs 26 verschiebbar ist (vgl. Fig. 2a), in der die Überwurfmutter 60 vollständig nach distal verschoben ist), mit dem distalen Ende 19 des Schafts 14 verschraubbar (vgl. 6b). Im verschraubten Zustand des Wechselkopfs 26 wird außerdem wegen der flach aufeinanderliegenden Flächen 48 und 50 ein Eindringen von Staub und Flüssigkeiten und sonstigen Kontaminationen in die Ankoppelstelle 30 zwischen dem Wechselkopf 26 und dem Schaft 14 vermieden.

Ferner ist zwischen dem Wechselkopf 26 und dem distalen Ende 19 des Schafts 14 ein elastisches Element 68 angeordnet, das bei einer Lockerung des Wechselkopfs 26 diesen vom distalen Ende 19 des Schafts 14 beabstandet. Das elastische Element 68 ist in dem gezeigten Ausführungsbeispiel in Form einer kleinen Druckfeder ausgebildet, die beispielhaft im distalen Ende 19 des Schafts 14 eingebettet ist. Bei einer Lockerung des Wechselkopfs 26 drückt das elastische Element 68 den Wechselkopf 26 bereits bei gelockerter Überwurfmutter 60 vom distalen Ende 19 des Schafts 14 weg, wodurch vermieden wird, daß bei einer Lokkerung der Überwurfmutter 60 der Wechselkopf 26 in engen Kontakt mit dem distalen Ende 19 des Schafts 14 verbleibt und der Benutzer somit keine Änderung der Bildinformation feststellen könnte. Damit jedoch vermieden wird, daß das elastische Element 68 bei vollständig gelöster Überwurfmutter 60 den Wechselkopf 26 unerwünschterweise vollständig abstößt, ist die Beabstandung des Wechselkopfs 26 durch das elastische Element 68 auf eine solche Länge begrenzt, die geringer ist als die Länge des Positionierstifts 52 bzw. 54, so daß auch bei vollständig entspanntem elastischem Element 68 noch eine Verbindung zwischen dem Wechselkopf 26 und dem Schaft 14 über die Positionierstifte 52 und 54 besteht, wodurch zumindest ein Abfallen des Wechselkopfs 26 vermieden werden kann.

Die zuvor erwähnten Positionierstifte 52 und 54 können in nicht dargestellten Ausgestaltungen auch zur elektrischen Signaloder Energieübertragung dienen.

In Fig. 2a) und b) sind ferner zwei Markierungen 62 und 64 dargestellt, die bei der Montage des Wechselkopfs 26 am Schaft 14 eine grobe Vororientierung des Wechselkopfs 26 ermöglichen, um das Einsetzen der Positionierstifte 52 und 54 in die Bohrungen 56 und 58 zu erleichtern.

Des weiteren sind die Positionierstifte 52 und 54 von dem Wechselkopf 26 abnehmbar, so daß sie im Falle eines Verbiegens oder einer sonstigen Beschädigung leicht ausgewechselt werden können.

Anstelle des Faserbündels 40 in dem Schaft 14 kann auch im Bereich des distalen Endes 19 des Schafts 14 ein elektronischer Bildaufnehmer (nicht dargestellt) hinter dem Deckglas 44 angeordnet sein, der über eine nach proximal führende Signalleitung mit einer Videomonitoreinheit verbunden ist. Die Abbildungsoptik 42 ist bei einer solchen Variante wiederum vollständig im Wechselkopf 26 oder teilweise im Wechselkopf 26 und teilweise im Schaft 14 angeordnet. Bei einer Lockerung des Wechselkopfs 26 liegt dann der Bildaufnehmer nicht mehr exakt in der Bildebene der Abbildungsoptik 42, wodurch eine wahrnehmbar veränderte Bildinformation nach proximal übertragen wird.

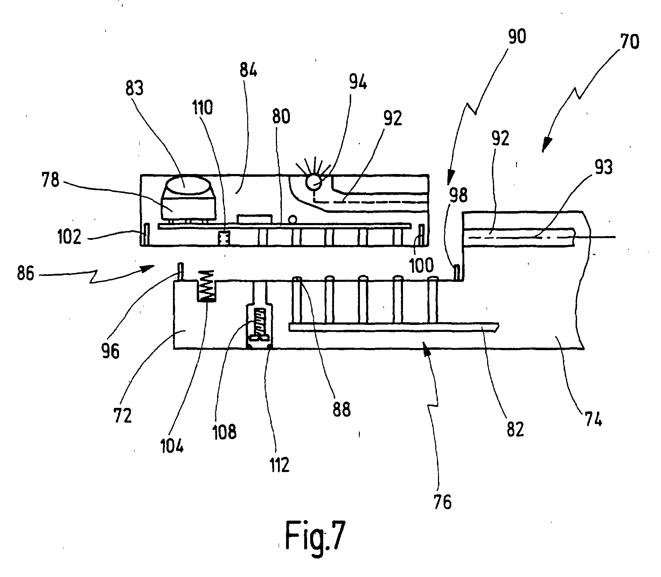

In Fig. 7 ist als weiteres Ausführungsbeispiel ein mit dem allgemeinem Bezugszeichen 70 bezeichnetes optisches Instrument im Bereich eines distalen Endes 72 seines Schafts 74 dargestellt.

Das optische Instrument 70 ist beispielsweise ein Endoskop, wobei der Schaft 74 beispielsweise starr ist.

Dieses optische Instrument 70 weist ein Übertragungssystem 76 zum Übertragen von Bildinformation von distal nach proximal auf, das einen optoelektronischen Bildaufnehmer (Videobildsensor) 78 aufweist, dessen Bildinformation in Form elektrischer Signale über elektrische Signalleitungen 80 und 82 nach proximal geleitet werden.

Der optoelektronische Bildaufnehmer 78, dem eine Abbildungsoptik 83 vorgeschaltet ist, ist jedoch nicht im Schaft 74 des Instruments 70 angeordnet, sondern in einem Wechselkopf 84, der von dem Schaft 74 abnehmbar ist. Die elektrischen Signalleitungen 80 und 82 gehen in mit dem Schaft 74 verbundenen Zustand des Wechselkopfes 84 durch eine Ankoppelstelle 86 hindurch, wobei die Ankoppelstelle 86 entsprechend mit einer Mehrzahl von Kontakten 88 wechselkopfseitig und schaftseitig ausgestattet ist.

Der Bildaufnehmer 78 und die Abbildungsoptik 83 können auch als vollständige Kamera-Einheit ausgebildet sein.

Bei einer Lockerung des Wechselkopfs 84 werden die Kontakte 88 entsprechend geöffnet und dadurch die Signalleitung durch die elektrischen Signalleitungen 80 und 82 entsprechend unterbrochen, so daß keine Bildinformation mehr vom Bildaufnehmer 78 nach proximal übertragen werden kann und somit das Bild für den Beobachter verschwindet.

Das Instrument 70 weist weiterhin ein Übertragungssystem 90 zum Übertragen von Beleuchtungsleistung von proximal nach distal auf, das wiederum durch einen Lichtleiter 92 in Form eines Faserbündels gebildet ist, das im mit dem Schaft 74 verbundenen Zustand des Wechselkopfs 84 durch die Ankoppelstelle 86 hindurchgeht.

Anstelle eines lichtleitenden Übertragungssystems zur Übertragung von Beleuchtungsleistung kann ein solches jedoch auch auf einer elektrischen Energieleitung 93 beruhen, die im Bereich der Ankoppelstelle 86 im Falle einer Lockerung des Wechselkopfs 84 unterbrochen wird, wobei dann im Wechselkopf 84 eine Lichtquelle 94, beispielsweise in Form einer LED, angeordnet ist, die beim Lockern des Wechselkopfs 84 entsprechend erlischt, wodurch eine wahrnehmbare Verdunklung des übertragenen Bildes auftritt.

Des weiteren sind bei dem in Fig. 7 dargestellten Ausführungsbeispiel wiederum Positionierstifte 96 und 98 vorgesehen, die in entsprechende Bohrungen 100 und 102 im Wechselkopf 84 eingreifen, um eine exakte Positionierung des Wechselkopfs 84 bezüglich des distalen Endes 72 des Schafts 74 und damit eine exakte Schließung der Kontakte 88 zwischen dem Schaft 74 und dem Wechselkopf 84 zu gewährleisten.

Ferner ist wiederum ein elastisches Element 104 vorgesehen, das bei Lockerung des Befestigungselements 108 in der Verankerung 110 den Wechselkopf 84 beabstandet, so daß die Bildinformation durch Öffnen mindestens eines Kontaktes merkbar verändert wird. Das Befestigungselement 108 ist durch einen Ring 112 verliergesichert.

In diesem Ausführungsbeispiel wurde die Orientierung der Flächen der Ankoppelstelle 86 willkürlich gewählt.

## Patentansprüche

1. Optisches Instrument, insbesondere Endoskop (12), mit einem Schaft (14) und mit einem wechselkopf (26), der mit dem distalen Ende (19) des Schafts (14) an einer Ankoppelstelle (30) lösbar verbunden ist, weiterhin mit einem ersten übertragungssystem (32) zum Übertragen von Beleuchtungsleistung nach distal und mit einem zweiten Übertragungssystem (38) zum Übertragen von Bildinformation nach proximal, wobei das erste Übertragungssystem (32) und das zweite übertragungssystem (38) durch die Ankoppelstelle (30) hindurchgehen, wobei das zweite Übertragungssystem eine Abbildungsoptik (42) aufweist, wobei die Abbildungsoptik (42) teilweise im Wechselkopf (26) und teilweise im Schaft (14) angeordnet ist, und der Wechselkopf (26) und/oder die Ankoppelstelle (30) derart ausgebildet ist, daß bei einer Lockerung des wechselkopfs (26) durch das zweite Übertragungssystem (38) eine qualitativ wahrnehmbar veränderte Bildinformation übertragen wird, **dadurch gekennzeichnet, dass** der im Schaft (14) angeordnete Teil der Abbildungsoptik (42) auswechselbar ist.

2. Instrument nach Anspruch 1, **dadurch gekennzeichnet, daß** das erste übertragungssystem (32) einen Lichtleiter umfaßt, der sich durch den Schaft (14) und durch den wechselkopf (26) erstreckt, und an der Ankoppelstelle (30) unterbrochen ist, und daß das distale Ende (19) des Schafts (14) und der Wechselkopf (26) an der Ankoppelstelle (30) jeweils eine ebene, vorzugsweise polierte Fläche (48, 50) aufweisen.

3. Instrument nach Anspruch 1, **dadurch gekennzeichnet, daß** das erste übertragungssystem zumindest eine Lichtquelle und eine elektrische Energieleitung von proximal zur Lichtquelle aufweist, wobei die Lichtquelle im Wechselkopf angeordnet ist, und daß die Ankoppelstelle so ausgebildet ist, daß bei einer Lockerung des Wechselkopfes die Energieleitung nach distal unterbrochen ist.

4. Instrument nach einem der Ansprüche 1 bis 3, **dadurch gekennzeichnet, daß** der Wechselkopf (26) mit dem distalen Ende (19) des Schafts (14) an der Ankoppelstelle (30) mittels zumindest eines Positionierstifts (52, 54), der in eine entsprechende Bohrung (56, 58) eingreift, verbunden ist.

5. Instrument nach Anspruch 4, **dadurch gekennzeichnet, daß** der zumindest eine Positionierstift (52, 54) am Wechselkopf (26) und die Bohrung (56, 58) am distalen Ende (19) des Schafts (14) vorgesehen ist.

6. Instrument nach Anspruch 4 oder 5, **dadurch gekennzeichnet, daß** der zumindest eine Positionierstift (52, 54) auswechselbar ist.

7. Instrument nach einem der Ansprüche 4 bis 6, **dadurch gekennzeichnet, daß** der zumindest eine Positionierstift zur elektrischen Signal- oder Energieübertragung dient.

8. Instrument nach einem der Ansprüche 1 bis 7, **dadurch gekennzeichnet, daß** zwischen dem Wechselkopf (26) und dem distalen Ende (19) des Schafts (14) ein elastisches Element (68) angeordnet ist, das bei einer Lockerung des Wechselkopfs (26) diesen vom distalen Ende (19) des Schafts (14) beabstandet.

9. Instrument nach Anspruch 8 und einem der Ansprüche 4 bis 7, **dadurch gekennzeichnet, daß** die Beabstandung auf weniger als die Länge des zumindest einen Positionierstifts (52, 54) begrenzt ist.

10. Instrument nach einem der Ansprüche 1 bis 9, **dadurch gekennzeichnet, daß** in dem wechselkopf zumindest ein Arbeitselement angeordnet ist.

11. Instrument nach Anspruch 10, **dadurch gekennzeichnet, daß** das zumindest eine Arbeitselement mittels eines Steckers mit dem distalen Ende des Schafts verbindbar ist.

## Claims

1. An optical instrument, in particular an endoscope (12), comprising a shaft (14) and an interchangeable head (26) that is detachably connected to the distal end (19) of the shaft (14) at a coupling point (30), further comprising a first transmission system (32) for transmitting illuminating power in distal direction and a second transmission system (38) for transmitting image information in proximal direction, the first transmission system (32) and the second transmission system (38) passing through the coupling point (30), the second transmission system having an imaging optics (42), the imaging optics (42) being arranged partially in the interchangeable head (26) and partially in the shaft (14), the interchangeable head (26) and/or the coupling point (30) being designed in such a way that upon loosening of the interchangeable head (26) image information of perceptively modified quality is transmitted by the second transmission system (38), **characterized in that** the part of the imaging optics (42) arranged in the shaft (14) being exchangeable.

2. The instrument of claim 1, **characterized in that** the first transmission system (32) comprises an optical waveguide that extends through the shaft (14) and through the interchangeable head (26), and is interrupted at the coupling point (30), and the distal end (19) of the shaft (14) and the interchangeable head (26) each have a flat, preferably polished surface (48, 50) at the coupling point (30).

3. The instrument of claim 1, **characterized in that** the first transmission system has at least one light source and an electric power line from proximally to the light source, the light source being arranged in the interchangeable head, and wherein the coupling point is designed such that upon loosening of the interchangeable head the power line is interrupted in the distal direction.

4. The instrument of anyone of claims 1 through 3, **characterized in that** the interchangeable head (26) is connected to the distal end (19) of the shaft (14) at the coupling point (30) by means of at least one positioning pin (52, 54) that engages in a corresponding bore (56, 58).

5. The instrument of claim 4, **characterized in that** the at least one positioning pin (52, 54) is provided on the interchangeable head (26), and the bore (56, 58) is provided on the distal end (19) of the shaft (14).

6. The instrument of claim 4 or 5, **characterized in that** the at least one positioning pin (52, 54) is exchangeable.

7. The instrument of anyone of claims 4 through 6, **characterized in that** the at least one positioning pin serves the purpose of transmitting electric signals or electric power.

8. The instrument of anyone of claims 1 through 7, **characterized in that** there is arranged between the interchangeable head (26) and the distal end (19) of the shaft (14) an elastic element (68) that upon loosening of the interchangeable head (26) distances the latter from the distal end (19) of the shaft (14).

9. The instrument of claim 8 and anyone of claims 4 through 7, **characterized in that** the distancing is limited to less than the length of the at least one positioning pin (52, 54).

10. The instrument of anyone of claims 1 through 9, **characterized in that** at least one working element is arranged in the interchangeable head.

11. The instrument of claim 10, **characterized in that** the at least one working element can be connected to the distal end of the shaft by means of a plug.

## Revendications

1. Instrument optique, plus particulièrement un endoscope (12), avec une tige (14) et avec une tête interchangeable (26) reliée de manière amovible à l'extrémité distale (19) de la tige (14) au niveau d'un raccordement (30), en outre avec un premier système de transmission (32) pour transmettre une puissance d'éclairage vers le côté distal et avec un deuxième système de transmission (38) pour transmettre une information d'image vers le côté proximal, le premier système de transmission (32) et le deuxième système de transmission (38) traversant le raccordement (30), le deuxième système de transmission (38) comprenant une optique de reproduction (42), l'optique de reproduction (42) se trouvant en partie dans la tête interchangeable (26) et en partie dans la tige (14), et la tête interchangeable (26)et/ou le raccordement (30) étant conçus de telle sorte que, lors d'un desserrage (26) de la tête interchangeable (26), une information d'image modifiée perceptible de manière qualitative est transmise, **caractérisé en ce que** la partie de l'optique de reproduction (42) disposée dans la tige (14) est interchangeable.

2. Instrument selon la revendication 1, **caractérisé en ce que** le premier système de transmission (32) comprend un conducteur optique qui traverse la tige (14) et la tête interchangeable (26) et qui est interrompu au niveau du raccordement (30), et **en ce que** l'extrémité distale (19) de la tige (14) et la tête interchangeable (26) comprennent, au niveau du raccordement (30), une surface plane, de préférence polie (48, 50).

3. Instrument selon la revendication 1, **caractérisé en ce que** le premier système de transmission comprend au moins une source de lumière et une ligne d'alimentation électrique du côté proximal vers la source de lumière, la source de lumière étant disposée dans la tête interchangeable, et **en ce que** le raccordement est conçu de telle sorte que, lors d'un desserrage de la tête interchangeable, la ligne d'alimentation vers le côté distal est interrompue.

4. Instrument selon l'une des revendications 1 à 3, **caractérisé en ce que** la tête interchangeable (26) est reliée à l'extrémité distale (19) de la tige (14) au niveau du raccordement (30) au moyen d'au moins une broche de positionnement (52, 54) qui s'emboîte dans un alésage (56, 58) correspondant.

5. Instrument selon la revendication 4, **caractérisé en ce que** la broche de positionnement (52, 54), au nombre d'une au moins, est disposée au niveau de la tête interchangeable (26) et l'alésage (56, 58) est disposé au niveau de l'extrémité distale (19) de la tige (14).

6. Instrument selon la revendication 4 ou 5, **caractérisé en ce que** la broche de positionnement (52, 54), au nombre d'une au moins, peut être interchangée.

7. Instrument selon l'une des revendications 4 à 6, **caractérisé en ce que** la broche de positionnement, au nombre d'une au moins, sert à la transmission électrique de signaux ou d'énergie.

8. Instrument selon l'une des revendications 1 à 7, **caractérisé en ce que**, entre la tête interchangeable (26) et l'extrémité distale (19) de la tige (14), est disposé un élément élastique (68) qui, lors d'un desserrage de la tête interchangeable (26), écarte celle-ci de l'extrémité distale (19) de la tige (14).

9. Instrument selon la revendication 8 et selon l'une des revendications 4 à 7, **caractérisé en ce que** l'écartement est limité à une distance inférieure à la longueur de la broche de positionnement (52, 54), au nombre d'une au moins.

10. Instrument selon l'une des revendications 1 à 9, **caractérisé en ce que**, dans la tête interchangeable, est disposé au moins un élément fonctionnel.

11. Instrument selon la revendication 10, **caractérisé en ce que** l'élément fonctionnel, au nombre d'un au moins, peut être relié à l'extrémité distale de la tige à l'aide d'un connecteur.
